# EUROPEAN PATENT APPLICATION

(11) **EP 1 889 615 A2**
(43) Date of publication of application: **20.02.2008**
(21) Application number: 07113658.4
(22) Date of filing: 02.08.2007
(51) Int. Cl.: A61K 31/352, A61K 31/7008, A61P 19/02, A61P 43/00

(54) **Formulations for oral administration with a health promoting effect on the osteoarticular and muscleskeleton apparatus**

(30) Priority: 04.08.2006 IT TO20060580
(71) Applicant: ROTTAPHARM S.P.A., 20122 Milano (IT)
(72) Inventor: Senin, Paolo, 20052, MONZA (Milano) (IT); Setnikar, Ivo, 20146, MILANO (IT); Rovati, Luigi Angelo, 20052, MONZA (Milano) (IT)
(74) Representative: Rambelli, Paolo

(57) **Abstract**

A pharmaceutical composition or dietary supplement or another preparation suitable for oral administration, having health promoting effects on the joints and the musculoskeletal apparatus, comprising glucosamine or a simple or mixed salt thereof, preferably in the form of crystalline glucosamine sulphate, and mangostin, preferably in the form of an extract of *Garcinia mangostana* fruit. Thanks to a combination of activities, such as that of stimulating trophism and protecting joint cartilage and an anti-inflammatory activity towards musculoskeletal tissues, the association of the aforementioned active agents may be used to obtain health promoting effects for maintaining osteoarticular wellbeing and for the prevention and treatment of degenerative or inflammatory disorders of the osteoarticular or musculoskeletal apparatus.

## Description

The subject of the present invention consists of preparations for oral administration, in the form of tablets, capsules, powders for extemporaneous use or a liquid, capable of exercising health-promoting effects on the osteoarticular and musculoskeletal apparatuses, thanks to a combination of activities, such as that of stimulating cartilage trophism, protecting cartilage and having anti-inflammatory effects on osteoarticular and musculoskeletal tissues, with consequent beneficial effects on osteoarticular functional efficiency and on disorders, such as for example osteoarthritis, leading to invalidating states associated with pain and restricted motion, more or less correlated with the state of advancement of the ongoing disease process.

### INTRODUCTION

Freedom of movement and the efficiency of the osteoarticular and musculoskeletal apparatuses are fundamental components for good quality of life, well understood by any unfortunate individuals who, as a result of trauma, nervous or musculoskeletal disorders, suffer from invalidity due to pain or restricted motion.

One osteoarticular disorder affecting a large number of individuals all over the world, interfering with their social lives, their socio-economic status and their physical and mental wellbeing is osteoarthritis (OA) [Carr 1999]. OA is a degenerative disease which gradually worsens with age and is due to attrition and wearing away of the joints, making them painful and restricting motion. OA strikes mostly in later life [Creamer *et al.* 1997] and the progressive ageing of the population has meant that OA has become one of major global healthcare problems.

OA is characterised by degeneration of the joint cartilage, which loses elasticity, softens and wears easily, with the appearance of cracks and loss of continuity, until eventually leading to exposure of the bone heads, conditions making movement difficult and painful.

Cartilage degeneration initiates slowly and is asymptomatic until the disease process has advanced to the stage where it is no longer reversible and causes pain and invalidity. Treatments at this stage are pharmacological and symptomatic in nature, for the relief of pain and to restrict the inflammatory processes of the joints affected by OA, but involve many, often serious, side effects. The drugs most often used include NSAIDs (Non-Steroidal Anti-Inflammatory Drugs) which act by inhibiting the cyclooxygenase family of enzymes (COX). The COX enzymes are involved in the arachidonic acid biochemical cascade pathway leading to the production of proinflammatory cytokines (prostaglandins, prostacyclins, thromboxanes and leukotrienes) responsible for the typical symptoms of inflammation, i.e. pain, hyperaemia, oedema and leukocyte activation. However, NSAIDs do not only inhibit the COX-2 members of the family, i.e. those induced by inflammatory processes, but also the constitutive COX-1 members, i.e. those normally present in tissues, generating prostaglandins with protective functions, such as the production of gastric mucus or the regulation of renal flux. Hence, the prolonged use of NSAIDs may lead to serious adverse effects and above all damage the mucosa of the gastro-enteric tract and renal function. Also, it has been known for some time [McKenzie *et al.* 1976] that NSAIDs accelerate the degenerative processes associated with OA and should therefore not be used for this disease. On the other hand, the alternative drugs (cortisones, high dose paracetamol etc.) are even more dangerous than NSAIDs, and do not permit prolonged treatment without serious side effects.

More recently, NSAIDs have been developed with more selective action towards COX-2 (meloxicam, rofecoxib, celecoxib, parecoxib, valdecoxib, etoricoxib), but it has also been observed that these do not possess absolute selectivity, and cause side effects similar to those of traditional NSAIDs. Furthermore, they increase the risks of cardiovascular thrombosis, and must thus be used with extreme caution.

For the above reasons, it is essential to resort to the early use of measures which impede the disease processes leading to OA, before they have the opportunity to manifest themselves clinically. These measures should guarantee and maintain the wellbeing and good function of the joints from a young age.

The main pathogenic factors leading to joint cartilage degeneration are represented by the metabolic deregulation of cartilage matrix proteoglycan biosynthesis and the action of endogenous substances which accelerate depolymerisation and catabolism. Indeed, the chemical and physical integrity of the proteoglycans confined in the three-dimensional collagen fibre network is essential for keeping joint cartilage turgid, elastic, smooth and free from lesions in continuity. A suitable lifestyle is essential for keeping joint cartilage healthy, in particular, moderate and regular physical activity, which does not however expose the cartilage to excessive trauma. It is also essential to have a proper dietary intake of the substances required by the body for proteoglycan biosynthesis, and finally, it is essential to prevent the formation of those substances which attack cartilage, causing its degradation.

Such conditions are not always easy to obtain, and it is precisely with this viewpoint that the research forming the subject of the present invention has been initiated, *i.e.* the identification of natural or naturally derived substances capable of being used as dietary supplements for aiding the aforementioned actions.

To this end, the active ingredients identified and selected following careful selection have been glucosamine, and the simple or mixed salts thereof, and mangostin, preferably in the form of *Garcinia mangostana* fruit extracts, for reasons of brevity referred to as GMFE. Glucosamine is a substrate for proteoglycan biosynthesis, and stimulates and regulates its anabolism. GMFE blocks the formation and the action of proinflammatory cytokines and the endogenously produced enzymes which attack cartilage and accelerate the degenerative processes.

The association of glucosamine with mangostin or GMFE produces synergic health promoting effects which can be demonstrated experimentally by *"in vitro"* and *"in vivo"* tests, the extraordinarily positive results of which, exceeding all expectations, are described in detail in the experimental section. Furthermore, thanks to the synergic action, the aforementioned effects are obtained at relatively low doses, acceptable within the regulations governing dietary supplements.

The synergic compositions forming the subject of the invention and the use thereof for the preparation of medicaments and/or dietary supplements and/or other preparations having the above-described health-promoting effects are defined in the Claims of this patent application.

### CHARACTERISTICS OF THE SUBSTANCES USED

### Glucosamine and the simple or mixed salts thereof

### General information

Glucosamine is 2-amino-2-deoxyglucose, a monosaccharide synthesised physiologically in the human body from glucose, and used in the synthesis of a number of the structural macromolecules of connective tissues, in particular joint cartilage.

In the composition forming the subject of the present invention, glucosamine is preferably present as crystalline glucosamine sulphate (**CGS**), also carrying the sulphate ions necessary for the biosynthesis of chondroitin sulphate and proteoglycans, fundamental structural components of the matrix of joint cartilage. **CGS** is a mixed salt, where glucosamine, sulphate ions, sodium ions and chloride ions are present stoichiometric proportions of 2:1:2:2. **CGS** is an original compound patented by Rotta Research Group (CGS RRG) in which the glucosamine is of natural origin, obtained from the acid hydrolysis of chitin, an organic component of the exoskeleton of crustaceans. This glucosamine, obtained from chitin, has identical biochemical and biological characteristics to the glucosamine produced endogenously in our bodies.

At pharmacological doses, CGS RRG has been used successfully to treat osteoarthritis. The relevant clinical studies are collected in the Cochrane Collection meta-analysis [Towheed *et al.* 2005] which have demonstrated the efficacy as a Symptomatic Slow Acting Drug for Osteoarthritis (SSADOA). More recent, long-term studies (3 years) have also shown its efficacy in arresting the progression of osteoarthritis, or rather, efficacy as a Disease Modifying Drug (DMD) [Reginster *et al.* 2001, Pavelka *et al.* 2002].

### Chondro-regenerative properties

**CGS** is the preferred and complete substrate for the biosynthesis of structural molecules of joint cartilage [Noyszewski *et al.* 2001] and is also a precursor of galactosamine, an aminosaccharide essential for the synthesis of keratin sulphate [Setnikar *et al.* 1986].

### Chondrostimulatory and chondroanabolic properties

CGS stimulates and normalises the synthesis of proteoglycans and chondroformation [Rodén 1956, Vidal y Plana 1980, Piperno *et al.* 2000, Bassleer *et al.* 1998]. It also increases the expression of aggrecan, a proteoglycan essential for the aggregation of proteoglycans to hyaluronic acid chains with the final formation of proteoglycan-aggregates (PA). PAs are gigantic molecules (40-100 millions of Daltons), confined, due to their size, in a three-dimensional collagen fibre network forming the molecular skeleton of the joint cartilage matrix. PAs absorb water avidly, which reswells them. The inability to escape from the three-dimensional collagen fibre network confers cartilage tissue with its rheological characteristics, namely the turgor, elasticity and plasticity necessary for performing its joint functions [Dodge *et al.* 2003].

### Chondroprotective and chondroreparatory properties

**CGS** inhibits the formation of various cytokines that damage cartilaginous structures. It inhibits the enzymes that attack and break down the macromolecules of the cartilage matrix such as collagenase, stromelysin and aggrecanase [Piperno *et al.* 2000].

### Dosage

The daily dose of glucosamine used within the scope of the invention is preferably 200-1000 mg (corresponding to 320-1600 mg of **CGS**) and preferably 250 mg (400 mg of **CGS**), the accepted daily dose for dietary supplements.

### Safety of use of CGS

The safety of use of the CGS used by Rottapharm for the present invention is guaranteed by thorough investigation of the acute, subacute and chronic experimental toxicology, male and female fertility, embryogenesis and embryotoxicity etc. and above all by the vast use it has had as a drug over tens of millions of documented patient-days of post-marketing research.

### Mangostin and Garcinia mangostana fruit extracts (GMFE)

### General information

*Garcinia mangostana* is a tropical tree from south-east Asian countries such as Thailand, India, Malaysia, Vietnam and the Philippines, and also Australia and Hawaii. Various GMFEs are used traditionally for topical application for the treatment of wounds and skin grazes and, orally, for infectious diarrhoea and urinary infections. More recently, GMFEs have attracted interest from western medicine and have been subjected to modem component identification techniques and pharmacological testing. Within the scope of said investigations, substances belonging mostly to the Xanthone family, a class of chemicals belonging to the polyphenols, derived from benzo-γ-pyrone, have been identified and isolated. Foremost among such substances is mangostin, namely 1,3,6-trihydroxy-7-methoxy-2,8-bis-(3-methyl-2-butenyl)-9H-xanthen-9-one, empirical formula C₂₄H₂₆O₆, m.w. 410.47 Da. Mangostin is used to standardise the titre of MGFEs.

Pharmacological studies conducted on MGFEs have identified the following activities.

### Antibacterial and antifungal activities

The xanthones contained in GMFE phytocomplexes have antibacterial and antifungal properties. In particular, α-mangostin and β-mangostin are active against *Mycobacterium tuberculosis,* with an MIC of 6.25 µg/ml [Suksamrarn *et al.* 2003] and methicillin and penicillin-resistant strains of *Staphylococcus aureus,* with an MIC di 1.6 µg/ml [Mahabusarakam *et al.* 1986, Inuma *et al.* 1996).

### Antioxidant activity

GMFEs possess considerable antioxidant activity, greater than that of vitamin E [Fan *et al.* 1997, Williams *et al.* 1994]. Of particular interest is the ability to inhibit low density lipoprotein (LDL) peroxidation, a chemical reaction which makes LDLs atherogenic and gives rise to arteriosclerosis [Williams *et al.* 1994).

### Antihistaminic and antiprostaglandin activity

GMFEs inhibit IgE mediated histamine release and A23187 stimulated prostaglandin E2 synthesis. They also inhibit passive cutaneous anaphylaxis in rats [Nakatani, Atsumi *et al.* 2002].

### Inhibition of the conversion of arachidonic acid into prostaglandin E2

In rat glioma C6 cells, GMFEs inhibit the conversion of arachidonic acid into prostaglandin E2, and hence the production of a potent inflammatory cytokine [Nakatani, Nakahata *et al.* 2002].

### Inhibition of cyclooxygenases

*In vitro,* GMFEs inhibit the cyclooxygenases induced by proinflammatory agents {COX-2), the enzymes responsible for the production of potent proinflammatory cytokines. This activity depends on the concentration of mangostin present in the extracts and is displayed with 50% inhibitory concentrations (IC₅₀) of 1-2 µM mangostin [Nakatani, Nakahata *et al.* 2002].

### Conclusions

By inhibiting the expression and the effects of proinflammatory cytokines, GMFEs exercise activities that are indispensable for maintaining joint wellbeing, so much so they are used in the USA as dietary supplements for joint health.

### Eman

Eman is dry *Garcinia mangostana* fruit extract, titred at 40% mangostin and the product of reference of the present invention, used in the experimental section.

### Eman dosage

The daily dose of Eman is preferably 12.5-450 mg (corresponding to 5-180 mg of mangostin) and preferably 75 mg of Eman (corresponding to 30 mg of mangostin).

### EXPERIMENTAL SECTION

The inventive target of the present patent application has been verified on a series of *"in vitro"* and *"in vivo"* pharmacological tests, evaluating and comparing the results in relation to the two active ingredients *i.e.* glucosamine (used in the form of **CGS**) and **Eman**, used individually or in association (**Ass**).

The chosen *"in vitro"* tests have been:
- On human blood: LPS stimulated production of IL-1, TNFα and IL-10.
- On human blood: LPS stimulated production of PGE2.
- On human blood: Coagulation-induced production of TXB2.
- On rabbit chondrocytes: IL-1β stimulated production of PGE2.
- On New Zealand white rabbit tibial joint cartilage explants in order to evaluate protective action in comparison to IL-1β induced cartilage degradation.

The chosen *"in vivo"* tests have been:
- The anti-inflammatory activities of **CGS, Eman** and **Ass** on zymosan-induced rat paw oedema.
- The analgesic activities of **CGS, Eman** and **Ass** according to Randall and Selitto [1957] on zymosan-induced rat paw hyperalgesia.

### Materials. Doses and Methods

### Materials

### Glucosamine

Pharmaceutical grade Crystalline Glucosamine Sulphate (**CGS**) has been used, a molecular complex in which glucosamine, sulphate ions, sodium and chloride ions are present in molar proportions equal to: 2:1:2:2. **CGS** has an m.w. of 573.30, a glucosamine content equal to 62.50% and glucosamine sulphate equal to 79.60%. Original CGS RRG has been used.

### Eman

The extract, in accordance with the previously specified characteristics, is normally available on the market (e.g. from HP Botanicals S.r.1., Milan).

### Ass (Association of CGS with Eman)

The daily doses of glucosamine and mangostin authorised and recommended in Italy for use as a dietary supplement are 250 mg and 30 mg respectively. This corresponds to 0.70 mmoles of **CGS** and 0.07 mmoles of mangostin. For pharmacological tests with the association of the two active ingredients, mixtures of **CGS** and **Eman** have been used so as to comply with a 10:1 molecular ratio between CGS and the mangostin present in Eman.

### Concentrations used "in vitro"

### • Eman

Solutions equal to mangostin concentrations of 0.1, 0.3, 1.0, 3.0, 10.0 and 30.0 µM (µmoles/1) have been used, indicated in the table as Man 0.1, Man 0.3, Man 1, Man 3, Man 10 and Man 30.

### • Crystalline glucosamine sulphate (CGS)

CGS concentrations of 1, 3, 10, 30, 100 and 300 µM (µmoles/1) have been used, indicated in the table as CGS 1, CGS 3, CGS 10, CGS 30, CGS 100, CGS 300.

### • Association (Ass)

As already specified, in order to comply with the recommended daily dose in humans, the molecular ratio between CGS and mangostin has been 10:1 and consequently the set of concentrations used has been Man 0.1 + CGS 1, Man 0.3 + CGS 3, Man 1 + CGS 10, Man 3 + CGS 30, Man 10 + CGS 100, Man 30 + CGS 300, indicated in the table as Ass 0.1, Ass 0.3, Ass 1, Ass 3, Ass 10 and Ass 30.

### • Lipopolysaccharide

1 µg/ml lipopolysaccharide from Escherichia coli (Sigma, Italy) has been used, indicated as LPS.

### • SB2 3580 (reference standard for LPS-inhibitory activity)

The set of concentrations 0.3, 1.0 and 3.0 µM has been used, indicated in the table as SB 0.3, SB 1 and SB 3.

### • CHX (Cycloheximide)

Cycloheximide is the reference standard for cartilage degrading enzyme inhibitory activity, and has been used at a concentration of 2.5 µg/ml.

### Doses used "in vivo"

In pharmacology, the diseases induced experimentally in animals are always dramatically and unnaturally more serious than those occurring spontaneously in humans. Thus, in experimental models, it is standard practice to use doses, expressed in mg/kg of body weight, much higher than those used for administration in humans. Furthermore, it is essential to consider that, in the extrapolation of doses from one animal species to another, it is more rational to refer to body surface area than to body weight [Guidance for Industry 2002]. If we consider reference to surface area, moving from rats to humans, it is necessary to divide the dose expressed in mg/kg by a factor of 6.2 [Guidance for Industry 2002]. Considering the above points and the daily doses used in humans and described in the formulation examples forming the subject of the present invention, the following doses, administered to rats orally, have been adopted in our experiments.

### • Eman

The doses have been equal to 3, 10 and 30 mg/kg of mangostin, indicated as Man 3, Man 10 and Man 30.

### Crystalline glucosamine sulphate (CGS)

The doses have been 40, 133 and 400 mg/kg indicated as CGS 40, CGS 133, CGS 400.

### • Association (Ass)

The doses have been Man 3 + CGS 40, Man 10 + CGS 133 and Man 30 + CGS 400 and indicated as Ass 3, Ass 10 and Ass 30.

### Methods

### 1. Human blood: LPS stimulated expression of IL-1, TNFα and IL-10.

Venous blood has been collected from healthy volunteers and treated with 0.38% Na citrate in order to prevent coagulation. 200 µl samples have been stimulated with 1 µg/ml of LPS for 24 hours at 37°C [Hartel *et al.* 2004], in the presence of the test products. The cytokines released into the plasma have been analysed using a multiple system for cytokines (Bio-Plex, Bio-Rad Laboratories, Milan, Italy) allowing the simultaneous determination of IL-1, TNFα and IL-10.

### 2. Human blood: LPS stimulate production of PGE2 (a COX-2 marker)

Human blood has been collected from health volunteers and made uncoagulable with 0.38% Na citrate. The test products have been added to 200 µl samples and then said samples stimulated with 100 µg/ml of LPS for 24 hours at 37°C. The PGE2 in the plasma, a marker of monocyte COX-2 activity [Panara *et al.* 1999], has been determined using a specific RIA method.

### 3. Human blood: Coagulation-induced thromboxanes production

The test products have been added to samples of human blood, collected from healthy volunteers, and then said samples allowed to coagulate by leaving to stand for 1 hour at 37°C. The thromboxane (TXB2) in the serum, a marker of platelet COX-1 activity (Panara *et al.* 1999], has been determined using a specific RIA method.

### 4. Rabbit chondrocytes: IL-1β-stimulated production of PGE2 (a COX-2 marker)

Joint cartilage chondrocytes, obtained from New Zealand white rabbits, have been cultured using the technique described by Berenbaum [1994]. Confluent cells have been stimulated for 24 hours with 1 ng/ml IL-1β. The test products have been added 20 minutes prior to stimulation. On completion of stimulation, the supernatant liquid has been collected and stored at -80°C until analysis. PGE2 in the culture medium has been measured using a radio-immunological method. The production of PGE2 in this model is a marker of the activity of the cyclooxygenases induced (COX-2) in cartilage from the proinflammatory stimulus represented by IL-1β.

### 5. Rabbit cartilage. Protective action against IL-β-induced cartilage degradation

Chondroprotective action has been assessed according to Sabatini et al. [2005] on tibial joint cartilage explants obtained from New Zealand white rabbits. The explants (2 x 2 mm) have been transferred to plates with wells and equilibrated for 48 hours at 37°C with high glucose DMEM plus 10% FCS and 50 µg/ml gentamycin. Explants have then been stimulated for 48 hours with 1 ng/ml IL-1β in aphenolic DMEM, 1 g/l glucose, 50 µg/ml gentamycin and 1% BSA. IL-1β elicits strong activation of the enzymes which depolymerise the proteoglycan aggregates of the cartilage matrix, resulting in its degradation, with the release of the breakdown products, mainly represented by the various glycosaminoglycans (GAG) making up the proteoglycan aggregates, into the culture liquid. Hence, the quantity of GAG released into the culture liquid is an indicator of the degradation of the macromolecules conferring joint cartilage with the turgor, elasticity and strength necessary for normal function [Farndale *et al.* 1982]. In order to assess the effects of the test products on IL-1β-induced cartilage degradation, they have been added to the cartilage explants 20 minutes prior to stimulation with IL-1β. Cartilage degradation has been assessed by expressing the GAG released into the culture liquid as a percentage of the total GAG measured following complete digestion of the cartilage explants using papain. As a reference standard, cycloheximide has been used, which, at a concentration of 2.5 µg/ml, blocks the synthesis of the degrading enzymes and hence degradation of the cartilage proteoglycan aggregates.

### 6. Rat. Anti-inflammatory activities in vivo

The anti-inflammatory activities of the test products have been assessed according to Di Rosa et al. [1971] on rat paw oedema induced by a subplantar injection of 4 mg di zymosan in 0.1 ml of isotonic saline. Paw volume has been measured pletismographically at 0, 1, 2, 4, 6 and 24 hours following the injection of zymosan, and expressed in ml of difference, with respect to the baseline volume. The test products, suspended in 5 ml/kg of 5% methylcellulose, have been administered with a gastric probe 30 minutes prior to the injection of zymosan. Celecoxib, a known COX-2 inhibitor, widely used clinically, and administered orally at a dose of 10 mg/kg, has been used as a reference standard. In order to express the treatment effects numerically, the areas under the curves of paw volume increase from 0 to 24 hours have been measured, thus giving the measurement of paw oedema in mlx24 hours.

### 7. Rat. Analgesic activities in vivo

The analgesic activities of the test products have been evaluated using the technique of Randall and Selitto [1957] on hyperalgesia induced in rat paw by a subplantar injection of 4 mg di zymosan in 0.1 ml of isotonic saline. The mechanical nociceptive threshold has been determined using an analgesimeter (Ugo Basile, Varese, Italy) at 0, 1, 2, 4, 6 and 24 hours following the injection of zymosan. The analgesimeter measures the weight (in grams) applied to the paw which causes the reflex retraction of the paw itself. The nociceptive threshold has been expressed as the difference (in grams) in nociceptive threshold at time 0. The test products have been administered orally 30 minutes prior to the injection of zymosan. Celecoxib, a known COX-2 inhibitor, widely used clinically as an analgesic-anti-inflammatory, and administered orally at a dose of 10 mg/kg, has been used as a reference standard. In order to express the treatment effects numerically, the areas under the curves of the analgesimetric weight increase on the inflamed paw have been measured from 0 to 24 hours, thus giving the measurement in g x 24 hours of the area under the analgesimetric curve.

### RESULTS

### 1. Human blood: Effects of the test substances on LPS stimulated IL-1 production

Interleukin 1 (IL-1) is one of the most aggressive proinflammatory cytokines. In joint cartilage, it inhibits the synthesis of proteoglycans and causes significant biochemical, morphological and mechanical damage [Sundy et al. 1999].

The effects of the test substances on the expression of IL-1 have been assessed using human blood, in which stimulation by LPS releases IL-1. The reference standard in this model is SB2 3580 (SB). SB has a 50% inhibitory concentration (IC₅₀) of 1.6 µM.

The results, reported in table 1, show that:
- **Eman** (expressed in the table as its mangostin content, abbreviated as "Man") inhibits the LPS-induced expression of IL-1 in a concentration dependent manner.
   The 50% inhibitory concentration (IC₅₀) is greater than 30 µM mangostin and may be estimated to be around 65 µM.
- **CGS** shows no appreciable inhibitory activity up to a concentration of 300 µM.
- **Ass.** Surprisingly, CGS at per se inactive concentrations, potentiates the IL-1-inhibitory activity of Eman, so much so that Ass 10 exerts 40% inhibition with 10 µM mangostin (Man 10), while the latter alone is weakly effective. Ass 30 (Man 30 + CGS 300) exerts 70% inhibition. The IC₅₀ in relation to mangostin is 22 µM. Thus CGS, ineffective alone, potentiates Man 3 fold in association.
- In conclusion, CGS, weakly active per se in inhibiting LPS-stimulated IL-1 release in human blood, surprisingly potentiates the inhibition of IL-1 release exerted by Eman.

### 2. Human blood. Effects of the test substances on LPS stimulated TNFα production

Tumour Necrosis Factor α (TNFα) is itself also a very aggressive proinflammatory cytokine, which, in joint cartilage, causes biochemical, morphological and mechanical damage [Sundy et al. 1999]. The effects of the test substances on TNFα expression has been assessed on human blood, where LPS stimulation releases TNFα. The reference standard in this model is SB2 3580 (SB), which has an IC₅₀ of 2.8 µM.

The results, reported in table 2, show that:
- **Eman** (expressed in the table as its mangostin content, abbreviated as "Man") inhibits TNFα expression in a concentration-dependent manner. The 50% inhibitory concentration (IC₅₀) is equal to 38 µM mangostin.
- **CGS** is weakly inhibitory, even at concentrations of 300 µM.
- **Ass.** CGS potentiates the TNFα-inhibitory activity of Eman. Indeed, Man 10 alone causes 15% inhibition of TNFα expression, however, when in association with CGS, it causes 37% inhibition (Ass 10). The IC₅₀ of Ass is equal to a mangostin concentration of 20 µM, potentiating Eman almost 2 fold.
- In conclusion, CGS, per se weakly active in inhibiting LPS-stimulated TNFα release in human blood, when in association with Eman, surprisingly potentiates the inhibition of TNFα release exerted by Eman.

### 3. Human blood. Effects of the test substances on LPS stimulated IL-10 production

Interleukin 10 (IL-10) is considered a chondroprotective cytokine because it positively modules the production and the effects of proinflammatory and chondrocyte damaging substances [Sundy et al. 1999]. Indeed, it:
a) inhibits the production of proinflammatory cytokines by macrophages,
b) inhibits TH1 cell differentiation,
c) inhibits the function of NK cells,
d) stimulates macrophage proliferation and function.

The effects of the test substances on LPS-stimulated IL-10 expression in human blood are reported in table 3. The reference standard has been SB2 3580 (SB), which stimulates IL-10 expression at a concentration of 0.3 µM.

The results, reported in table 3, show that:
- **Eman** (expressed in the table as its mangostin content, abbreviated as "Man") stimulates IL-10 expression at a concentration equal to 3 µM mangostin (Man 3). Stimulation is concentration-dependent and very pronounced. At a concentration of 30 µM mangostin, a 187% increase in IL-10 was detected with respect to baseline levels.
- **CGS** has no significant stimulatory or inhibitory activity at the concentrations tested (30, 100 and 300 µM).
- **Ass.** CGS surprisingly potentiates the stimulatory activity of Eman, practically doubling it. At the concentration of Man 30 (with 300 µM CGS), a 254% increase in IL-10 is observed.
- In conclusion, Eman exerts a powerful stimulatory action on the expression of the cytokine IL-10, a marker, among others, of chondroprotective effects. CGS exerts no appreciable stimulatory or inhibitory effects on IL-10 expression. However, in Ass, CGS surprisingly potentiates Eman in stimulating the expression of IL-10.

### 4. Human blood. Effects of the test substances on coagulation-induced production of thromboxane (a marker of platelet COX-1 activity)

In the blood coagulation process, the constitutive platelet cyclooxygenases (COX-1) release thromboxane (TXB2), and the effects on TXB2 release from coagulation are considered to be a marker of the COX-1 inhibitory or stimulatory activities [Panara et al. 1999].

The effects of the test substances on the production of TXB2 during coagulation are reported in table 4. The results indicate that:
- **Eman** (expressed in the table as its mangostin content, abbreviated as Man 1, 3 and 10) exerts weak inhibition on the release of TXB2, which is insignificant and not concentration-dependent.
- **CGS** has no significant stimulatory or inhibitory activity at concentrations of 10, 30, or 100 µM.
- **Ass.** The three associations studied (Ass 1, Ass 3 and Ass 10) exert no substantial stimulatory or inhibitory effects on TXB2 expression.
- In conclusion, Eman, CGS and associations thereof exert no effects on TXB2 release. Thus, it may be concluded that said products do not interfere with the constitutive cyclooxygenases (COX-1).

### 5. Human blood. Effects of the test substances on the inhibition of the production of PGE2 stimulated by 100 µg/ml LPS

The PGE2 appearing in human blood following stimulation with LPS is produced by the induced proinflammatory cyclooxygenases (COX-2) from monocytes. Hence, in this test, inhibitory activity towards PGE2 expression is a marker of anti-COX-2 activity, i.e. anti-inflammatory activity.

The effects of the test substances on LPS-stimulated PGE2 production in human blood are reported in table 5. The results indicate that:
- **Eman** (expressed in the table as its mangostin content, abbreviated as Man 1, 3 and 10) shows dose-dependent inhibition of LPS-stimulated PGE2 expression in human blood. The IC₅₀ is equal to 12.3 µM mangostin.
- **CGS** exerts no significant stimulatory or inhibitory activity at concentrations of 10, 30 or 100 µM.
- **Ass.** CGS significantly potentiates the inhibition of PGE2 expression exerted by Eman, so much so that the IC₅₀ of Ass corresponds to a concentration of 7.8 µM mangostin, with a 1.6 fold potentiation of the efficacy of non-associated Eman.
- In conclusion, Eman exerts concentration-dependent inhibition on LPS-induced PGE2 expression in human blood and markers of inducible proinflammatory cyclooxygenase activity (COX-2).

At concentrations of 10, 30 and 100 µM, CGS exerts no significant stimulatory or inhibitory effects on this marker, but surprisingly and unexpectedly potentiates the inhibitory effect of Eman.

### 6. Rabbit chondrocytes. Effects of the test substances on IL-1β stimulated PGE2 production

In rabbit chondrocytes, IL-1β induces activation of cyclooxygenases, COX-2, which is expressed through the production of PGE2, a proinflammatory cytokine, strongly damaging to cartilage.

The effects of the test substances on PGE2 production from COX-2 activation are reported in table 6. The results indicate that:
- **Eman** (expressed in the table as its mangostin content, abbreviated as "Man") at a concentration of 0.1 µM mangostin causes a 23% inhibition of IL-1β-induced PGE2 production. Inhibition reaches 50% at a concentration of 0.3 µM mangostin (Man 0.3) and 94% at a concentration of 3µM mangostin (Man 3). The concentration calculated to inhibit production of PGE2 by 50% (IC₅₀) is equal to 0.38 µM mangostin.
- **CGS** has no significant inhibitory or stimulatory activity at concentrations of 1, 3 or 10 µM.
- **Ass.** CGS in association with Eman (Ass 0.1, Ass 0.3 and Ass 1) exerts an inhibitory effect equal to that exerted by Eman when not in association with CGS. CGS does not interact with the PGE2-inhibitory activity of Eman.
- In conclusion, Eman exerts a potent inhibitory effect on COX-2 in cartilage which can be observed at a concentration of 0.1 µM mangostin. CGS is inactive at concentrations of 1, 3 and 10 µM. Eman in association with CGS is highly inhibitory thanks to the presence of Eman, however CGS does not interfere with the inhibitory activity of Eman.

In this model, which is highly representative of the damage caused to cartilage by a highly chondrocyte-damaging cytokine such as IL-1β, Eman exerts a very powerful anti-COX-2 protective effect, which can be observed at concentrations of 0.1 µM mangostin, which is unchanged when in association with CGS.

### 7. Protective effects of the test substances against IL-1β-induced cartilage degradation

In this test, the GAG released from cartilage explants into the culture liquid is measured following stimulation with a highly chondrocyte-damaging cytokine, such as IL-Iβ. GAG in the culture liquid is derived from the depolymerisation of the proteoglycan-aggregates confined within the three-dimensional collagen fibre network acting as the organic skeleton of the cartilage matrix. Hence, GAG in the culture liquid is a marker of cartilage degradation, due to the action of the destructive enzymes (metalloproteases and aggrecanases) activated by IL-1β.

The effects of the test substances on the IL-1β-stimulated release of GAG into the culture liquid are reported in table 7. The results indicate that:
1. Under baseline conditions, during the 48 hours of incubation, there has been degradation of 14% of the proteoglycan aggregates.
2. In cartilage explants, IL-1β induces activation of the enzymes causing degradation, increasing degradation to 69%.

- **Eman** (expressed in the table by its mangostin content, abbreviated as "Man") at concentrations of 0.1, 0.3 and 1.0 µM mangostin exerts no significant effects on IL-1β-induced GAG release.
- **CGS** at a concentration of 1 µM protects against IL-1β-induced cartilage degradation by 41 %. Protection reaches 64% and 77% at concentrations of 3 and 10 mM CGS respectively, with a 50% protective concentration (PC₅₀) estimated as 2.3 µM.
- **Ass.** Associations of CGS with Eman (Ass 0.1, Ass 0.3 and Ass 1) exert protective effects similar to that of non-associated CGS, with a PC₅₀ of approx. 2.2 µM CGS associated with Eman equal to 0.22 µM mangostin.
- In conclusion, CGS exerts a significant and potent inhibitory effect on cartilage degradation caused by IL-1β at concentration as low as 1 µM. 50% protection is achieved at a concentration of 2.3 µM CGS. Eman shows no significant activity towards affecting GAG release. The association of CGS with Eman is inhibitory thanks to the presence of CGS, and does not interfere with the inhibitory activity of CGS.

### 8. In vivo anti-inflammatory activities of the test substances in rats

The results obtained using the method of Di Rosa et al. [1971] are reported in table 8, showing the increased paw volume during the 24 hours following the subplantar injection of zymosan, measured from the area under the curve of the ml of oedema.
- **Eman** (expressed in the table by its mangostin content, abbreviated as "Man") at oral doses of 3, 10 and 30 mg/kg mangostin, inhibits oedema due to zymosan in a dose-dependent manner. This inhibition is significant with respect to controls, at a dose of 10 mg/kg (p < 0.05), with a percentage difference of 7% compared to controls. The percentage difference increases to 18% at a dose of 30 mg/kg and is much more significant (p < 0.0001).
- **CGS** at oral doses of 40, 133 and 400 mg/kg showed no significant anti-inflammatory activity in this experimental model.
- **Ass.** CGS, which in itself has no anti-inflammatory activity, in association with Eman significantly potentiates the activity, so as to make it significant at a dose of 3 mg/kg mangostin (Man alone has no significant effect at this dose). The Eman dose equal to 30 mg/kg mangostin (in association with 400 mg/kg CGS) becomes so effective as to exceed the efficacy of celecoxib at a dose of 10 mg/kg.
- In conclusion, Eman, at doses of 10 and 30 mg/kg mangostin, exerts a marked, significant and dose-dependent anti-inflammatory effect. CGS has practically no anti-inflammatory effect at the doses investigated (40, 133 and 400 mg/kg). Surprisingly however, CGS potentiates the anti-inflammatory effect of Eman, so much so that with an Eman dose equal to 30 mg/kg mangostin (in association with 400 mg/kg CGS) an anti-inflammatory effect comparable with that of 10 mg/kg celecoxib is observed.

### 9. Analgesic activities of the test substances in rats

Table 9 reports the areas under the curves of increased analgesimetric weight on the inflamed paw during the 24 hours following subplantar injection of zymosan.
■ **Eman** (expressed in the table by its mangostin content, abbreviated as "Man") at oral doses equal to 3, 10 and 30 mg/kg mangostin, exerts an analgesic effect in a dose-dependent manner. The effect is significant with respect to controls, at a dose of 10 mg/kg mangostin (p < 0.01), with a percentage difference of 35% compared to controls. The percentage difference increases to 48% at a dose of 30 mg/kg and is much more significant (p < 0.0001).
■ **CGS** at oral doses of 40, 133 and 400 mg/kg showed no significant analgesic activity in this experimental model.
■ **Ass.** CGS, which in itself has no analgesic activity, when in association with Eman significantly potentiates the analgesic effect, so much so as to make the activity of an Eman dose equal to 3 mg/kg mangostin significant (Eman itself is not significantly analgesic at this dose). The Eman dose of 30 mg/kg (in association with 400 mg/kg CGS) becomes so effective as to exceed the efficacy of celecoxib at a dose of 10 mg/kg.
■ In conclusion, Eman, at doses equal to 10 and 30 mg/kg mangostin, exerts a marked, significant and dose-dependent analgesic effect. CGS has practically no anti-inflammatory effect at the doses investigated (40, 133 and 400 mg/kg). Surprisingly however, it potentiates the analgesic effect of Eman, so much so that with an Eman dose equal to 30 mg/kg mangostin (in association with 400 mg/kg CGS) an analgesic effect grater than that of 10 mg/kg celecoxib is observed.

### 10. Conclusions

1. **Eman** has the following activities.

### a) Anti-inflammatory

Anti-inflammatory activity is exerted in vitro in various experimental models, and is probably correlated with the inhibitory activity of Eman towards COX-2 (cyclooxygenases induced by proinflammatory factors). On the other hand, Eman has no effect on the constitutive cyclooxygenases (COX-1) which have protective effects on the gastrointestinal mucosa and on the kidneys.

The anti-inflammatory activity of Eman has been confirmed in vivo on zymosan induced oedema in rats, where Eman has been administered orally.

### b) Chondroprotective

The chondroprotective activity of Eman is demonstrated in vitro by its capacity to inhibit the expression of chondrocyte damaging cytokines (IL-1, TNFα, PGE2) and its capacity to inhibit chondrocyte damage caused by IL-1β. Furthermore, Eman stimulates the expression of IL-10, a chondroprotective and anti-inflammatory cytokine.

### c) Analgesic

The analgesic activity of Eman is demonstrated in vivo in rats with the oral administration of Eman and applying an analgesimeter to the paw, made hyperalgesic by means of a subplantar injection of zymosan.

2. **Crystalline glucosamine sulphate (CGS)** has strong chondroprotective activity, inhibiting the depolymerisation of joint cartilage proteoglycan-aggregates induced by chondrocyte damaging cytokines such as IL-1β. The concentration of CGS with 50% activity is 2.3 µM.

CGS showed no significant effect in the other experimental models used in this study.

### 2. Association of Eman with crystalline glucosamine sulphate (Ass)

CGS, which, alone, only shows chondroprotective effect by inhibiting the depolymerisation of proteoglycan-aggregates, significantly, surprisingly and unexpectedly potentiates:
a) the inhibitory effect Eman exerts on the expression of proinflammatory and chondrocyte damaging cytokines (IL-1, TNFα, PGE2) ;
b) the inhibitory effect of Eman on the expression of PGE2, a COX-2 marker;
c) the stimulatory effect of Eman on the expression of IL-10, a cytokine which opposes the proinflammatory and chondrocyte damaging cytokines and which displays chondroprotective effects.

CGS also potentiates:
a) the anti-inflammatory activity of Eman in vivo;
b) the analgesic activity of Eman in vivo.

### EXAMPLES

The formulations reported hereinafter illustrate potential practical applications of the present invention, and as such, must not be taken to be limiting of the invention itself in any way.

Particular reference is made to the excipients of the various forms under consideration, which may be used alternatively to those explicitly mentioned in the present invention, in accordance with formulative-technological requirements, and which, in any case, may be selected from a vast range of products available on the market and well known to those skilled in the pharmaceutical sector, and hence of no inventive originality.

### Example 1: Tablets for oral use

The formulation reported (Table 10), concerns active ingredients and excipients (with a description of the corresponding technological function) that may be used in the preparation of tablets to be used for the oral administration of the subject of the present invention.

The daily dose of the active ingredients is incorporated in a single tablet, the preparation of which only requires standard operations, well known to anyone skilled in the art, i.e.:
- Weighing out of the individual components of the formulation.
- Wet granulation, by means of an aqueous solution of polyvinylpyrrolidone K-25, CGS and microcrystalline cellulose (Granulate A) in a suitable granulator.
- Drying of Granulate A in a convector oven at a temperature no greater than 50°C to constant weight (any other type of drying may be used providing it is previously validated).
- Sieving of Granulate A and the other components of the formulation.
- Preparation of the mixture for compression by homogenisation of Granulate A with the other components of the formulation in a suitable mixer.
- Compression of the resultant mixture in a suitable automated press, with the eventual attainment of tablets of suitable shape and size.

**Table 10. Example formulation in tablet form**

| **Active ingredients** | **Quantity/tablet** |
|---|---|
| Dried Garcinia mangostana fruit extract^{(*)} | 75 mg |
| *(Corresponding to mangostin)* | *(30 mg)* |
| Crystalline glucosamine sulphate | 400 mg |
| *(Corresponding to glucosamine)* | *(250 mg)* |

| | |
|---|---|
| (*) Contains 40% mangostin | |

| **Excipients** | **Quantity/tablet** | **Role** |
|---|---|---|
| Microcrystalline cellulose^{(*)} | 31.6 mg | Diluent/Disintegrant |
| Polyvinylpyrrolidone K-25^{(*)} | 11.8 mg | Binder |
| Cross-linked sodium carboxymethylcellulose^{(*)} | as required | Disaggregant |
| Talc^{(**)} | as required | Lubricant/Anti-adhesion agent |
| Mg stearate | as required | Lubricant/Anti-adhesion agent |
| Water^{(***)} | as required | Formulative support |

| | | |
|---|---|---|
| (*) Used in the preparation of the GSC granulate (granulate A) (**) The absolute and relative quantities of said excipients depend on the size and shape of the compression die, the type of press and the system for loading the powders into the compression chamber installed inside (***)Water is used in the preparation of granulate A and the amount in relation to the components of the granulate itself depends on the type and size of the granulator used. This is completely eliminated during drying. | | |

### Example 2: Capsules for oral use

The formulation reported (Table 11), concerns active ingredients and excipients (with a description of the corresponding technological function) that may be used in the preparation of capsules to be used for the oral administration of the subject of the present invention.

The daily dose of the active ingredients is incorporated in a single capsule, the preparation of which only requires standard operations, well known to anyone skilled in the art, i.e.:
- weighing out of the individual components of the formulation
- preparation of the mixture to be encapsulated by dry homogenisation in a suitable mixer
- Automated filling into a hard gelatine capsule of suitable size and desired colour.

**Table 11. Example formulation in capsule form**

| **Active ingredients** | **Quantity/capsule** |
|---|---|
| Dried Garcinia mangostana fruit extract^{(*)} | 75 mg |
| *(Corresponding to mangostin)* | *(30 mg)* |
| Crystalline glucosamine sulphate | 400 mg |
| *(Corresponding to glucosamine)* | *(250 mg)* |

| | |
|---|---|
| (*) Contains 40% mangostin | |

| **Excipients** | **Quantity/capsule** | **Role** |
|---|---|---|
| Microcrystalline cellulose^{(*)} | as required | Diluent/Disintegrant |
| Talc^{(*)} | as required | Lubricant/Anti-adhesion agent |
| Mg stearate^{(*)} | as required | Lubricant/Anti-adhesion agent |

| | | |
|---|---|---|
| (*)The absolute and relative quantities of the excipients depend on the size of the capsules used as administration vehicle and the measuring type/system installed in the filler used for batching the formulation into capsules. | | |

### Example 3: Powder for extemporaneous suspension for oral use, in thermosealed sachets

The formulation (Table 12) concerns active ingredients and excipients (with the description of the corresponding technological function) that may be used in the formulation of a powder for the preparation of extemporaneous suspensions to be taken orally. The daily dose is incorporated in a single portion of powder, contained in a thermosealed sachet consisting of an outer layer of paper, an intervening layer of aluminium and an inner layer of polyethylene.
The preparation operations of the pharmaceutical form in question, well known and applied by anyone skilled in the art, are essentially the following:
- weighing out and sieving of the individual components of the formulation
- preparation of the mixture of components by dry homogenisation in a suitable mixer
- thermoforming of the sachets on a suitable automated line
- filling and sealing of the sachets on said above mentioned automated line to give sachets with the desired shape and sizes containing the monodose powder for extemporaneous use.

**Table 12. Example of a powder formulation for extemporaneous suspension from sachets**

| **Active ingredients** | **Quantity/sachet** |
|---|---|
| Dried Garcinia mangostana fruit extract^{(*)} | 75 mg |
| *(Corresponding to mangostin)* | *(30 mg)* |
| Crystalline glucosamine sulphate | 400 mg |
| *(Corresponding to glucosamine)* | *(250 mg)* |

| | |
|---|---|
| (*) Contains 40% mangostin | |

| **Excipients** | **Quantity/sachet** | **Role** |
|---|---|---|
| Sorbitol^{(*)} | as required | Diluent/Sweetener |
| Citric acid^{(*)} | as required | Flavour enhancer |
| Polyethylene glycol 4000^{(*)} | as required | Lubricant/Plasticiser |
| Others^{(**)} | as required | Sweeteners/Flavourings |

| | | |
|---|---|---|
| (*) The absolute and relative quantities of said excipients depend on the type of sachet filler used and the sachet sizes. (**)Flavourings and sweeteners may be added freely, depending on the organoleptic preferences. | | |

### BIBLIOGRAPHY

1. Bassleer CT, Combal J-PA, Bougaret S, Malaise M. Effects of chondroitin sulphate and interleukin-1β on human articular chondrocytes cultivated in clusters. Osteoarthritis Cartilage 1998; 6:196-204.
2. Berenbaum F, Thomas CGS, Poiraudeau S, Béréziat G, Corvol MT, Masliah J. Insulin-like growth factors counteract the effect of interleukin-1β on type-II phospholipase A2 expression and arachidonic acid release by rabbit articular chondrocytes. FEES Lett. 1994; 340:51-55.
3. Carr AJ. Beyond disability: measuring the social and personal consequences of osteoarthritis. Osteoarthritis Cartilage 1999; 7:230-238.
4. Carr AJ. Beyond disability: measuring the social and personal consequences of osteoarthritis. Osteoarthritis Cartilage 1999; 7:230-238.
5. Creamer P, Hochberg MC. Osteoarthritis. Lancet 1997; 350:503-509.
6. Di Rosa M, Willoughby DA. Screens for anti-inflammatory drugs. J. Pharm. Pharmacol. 1971; 23,297.
7. Fan CT, De Su (1997).
8. Farndale RW, Sayers CA, Barrett AJ. A direct spectrophotometric microassay for sulphated glycosaminoglycans in cartilage cultures. Conn. Tiss. Res. 1982; 9:247-248.
9. Garcinia mangostana Chem. Pharm. Bull. (Tokyo) 51 (7) 857-859.
10. Guidance for Industry and reviewers. Estimating safe starting dose in clinical trials. FDA, CDER 2002.
11. Hartel C, von Puttkamer J, Gallner F, Strunk T, Schultz C. Dose-dependent immunomodulatory effects of acetylsalicylic acid and indomethacin in human whole blood: potential role of cyclooxygenase-2 inhibition. Scand. J. Immunol. 2004;60: 412-420.
12. linuma M, Tosa H, Tanaka T, Asai F et al (1996).
13. McKenzie LS, Horsburgh BA, Gosh P, Taylor TKF: Osteoarthrosis: uncertain rationale for anti-inflammatory drug therapy. Lancet, 1976; 1:980.
14. Mahabusarakam W, Wiriyachitra P, Phongpaichit S (1986).
15. Methicillin-resistant Staphylococcus aureus J Pharm Pharmacol 48(8) 861-865.
16. Nakatani K, Atsumi M, Arakawa T, Oosawa K, Shimura S, Nakahata N, Ohizumi Y. Inhibition of histamine release and prostaglandin E2 synthesis by mangosteen, a Thai medicinal plant. Biol Pharm Bull 2002; 25:1137-1141.
17. Nakatani K, Nakahata N, Arakawa T, Yasuda H, Ohizumi Y. Inhibition of cyclooxygenase and prostaglandin E2 synthesis by gamma-mangostin, a xanthone derivative in mangosteen, in C6 rat glioma cells. Biochem Pharmacol 2002; 63:73-79.
18. Noyszewski EA, Wroblewski K, Dodge GR, Kudchodkar S, Beers J, Sarma AVS, Reddy R. Preferential incorporation of glucosamine into the galactosamine moieties of chondroitin sulphates in articular cartilage explants. Arthritis Rheum. 2001; 44:1089-1095.
19. Panara MR, Renda G, Sciulli MG, Santini G, Di Giamberardino M, Rotondo MT, Tacconelli S, Seta F, Patrono C, Patrignani P. Dose-dependent inhibition of platelet cyclooxygenase-1 and monocyte cyclooxygenase-2 by meloxicam in healthy subjects. J Pharmacol Exp Ther 1999; 290:276-280.
20. Pavelkà K, Gatterova J, Olejarova M, Machacek S, Giacovelli G, Rovati LC. Glucosamine sulphate use and delay of progression of knee osteoarthritis: a 3-year, randomised, placebo-controlled, double-blind study. Arch Intern Med 2002; 162:2113-2123.
21. Piperno M, Reboul P, Hellio Le Graverand MP, Peschard MJ, Annefeld M, Richard M, Vignon E. Glucosamine sulphate modulates dysregulated activities of human osteoarthritic chondrocytes in vitro. Osteoarthritis Cartilage 2000; 8:207-212.
22. Randall LO, Selitto JJ. A method for measurement of analgesic activity on inflamed tissue. Arch. Int. Pharmacodyn. 1957; 111:409.
23. Reginster JY, Deroisy R, Rovati LC, Lee RL, Lejeune E, Bruyere O, Giacovelli G, Henrotin Y, Dacre JE, Gossett C. Long-term effects of glucosamine sulphate on osteoarthritis progression: a randomised, placebo-controlled clinical trial. Lancet 2001; 357:251-256.
24. Roden L. Effect of hexosamines on the synthesis of chondroitin sulphuric acid in vitro. Arkiv for Kemi 1956; 10:345-352.
25. Sabatini M, Lesur C, Thomas M, Chomel A, Anract P, de Nanteuil G, Pastoureau P. Effect of inhibition of matrix metalloproteinases on cartilage loss in vitro and in a guinea pig model of osteoarthritis. Arthritis Rheum. 2005; 52, 171-180.
26. Sandy JD, Gamett D, Thompson V, Verscharen C. Chondrocyte-mediated catabolism of aggrecan: aggrecanase-dependent cleavage induced by interleukin-1 or retinoic acid can be inhibited by glucosamine. Biochem J 1998; 335:59-66.
27. Setnikar I, Giachetti C, Zanolo G. Pharmacokinetics of glucosamine in the dog and in man. ArzneimittelForschung 1986; 36:729-735.
28. Suksamrarn S, Suwannapoch N, Phakhodee W, Thanuhiranlert J et al. (2003).
29. Sundy JS, Patel DD, Haynes BF. Cytokines and cytokine receptors. In: Gallin JI, Snyderman R eds. Inflammation basic principles and clinical correlates. Lippincott Philadelphia 1999 Appendix B, pages 1303-1308.
30. Towheed TE, Anastassiades TP, Shea B, Houpt J, Welch V, Hochberg MC. Glucosamine therapy for treating osteoarthritis (Cochrane Review). In: The Cochrane Library, Issue 2, 2001. Oxford: Update Software.
31. Vidal y Plana RR, Karzel K. Glukosamin. Seine Bedeutung für den Knorpelstoffwechsel der Gelenke. Biochemie der Proteoglycane. Untersuchungen auf in vitro-Kulturen embryonaler Mäuse-Fibroblasten and Knochenanlagen. Fortschr Med 1980;98:555-562.
32. Volpi N. Oral absorption and bioavailability of ichthyc origin chondroitin sulphate in healthy male volunteers. Osteoarthritis Cartilage 2003; 11:433-441.
33. Williams P, Ongsakul M, Proudfoot J, Croft K, Beilin L (1994) Mangostin inhibits the oxidative modification of human low density lipoprotein. Free Rad Res 23:175-184.

### TABLES

**Table 1. Human blood. Expression of IL-1 stimulated by 1 µg/ml LPS Mean and SD. N = 8**

| Product µM | IL-1 ng/ml | SD ng/ml | %Diff. vs control | IC₅₀ µM |
|---|---|---|---|---|
| Control | 4.55 | 0.28 | 0.0 | |
| | | | | |
| Man 3 | 4.74 | 0.18 | 4.1 | |
| Man 10 | 4.49 | 0.20 | -1.3 | 65 |
| Man 30 | 3.06 | 0.20 | -32.7 | |
| | | | | |
| CGS 30 | 4.65 | 0.20 | 2.1 | |
| CGS 100 | 4.72 | 0.19 | 3.6 | N/A |
| CGS 300 | 4.69 | 0.21 | 3.1 | |
| | | | | |
| ASS 3 | 4.51 | 0.22 | -0.9 | |
| ASS 10 | 2.75 | 0.19 | -39.6 | 22 |
| ASS 30 | 1.36 | 0.17 | -70.2 | |
| | | | | |
| SB 0.3 | 3.44 | 0.40 | -24.5 | |
| SB 1 | 2.48 | 0.24 | -45.6 | 1.6 |
| SB 3 | 1.35 | 0.15 | -70.4 | |

**Table 2. Human blood. Expression of TNFα stimulated by 1 µg/ml LPS Mean and SD. N = 8**

| Product µM | TNFα ng/ml | SD ng/ml | %Diff. vs control | IC₅₀ µM |
|---|---|---|---|---|
| Control | 15.96 | 2.45 | 0.0 | |
| | | | | |
| Man 3 | 15.65 | 2.50 | -2.0 | |
| Man 10 | 13.60 | 2.40 | -14.8 | 38 |
| Man 30 | 7.06 | 0.96 | -55.8 | |
| | | | | |
| CGS 30 | 14.70 | 2.25 | -7.9 | |
| CGS 100 | 13.80 | 2.35 | -13.5 | >300 |
| CGS 300 | 12.70 | 2.22 | -20.4 | |
| | | | | |
| ASS 3 | 13.80 | 2.50 | -13.5 | |
| ASS 10 | 10.07 | 2.40 | -36.9 | 20 |
| ASS 30 | 5.89 | 0.96 | -63.1 | |
| | | | | |
| SB 0.3 | 12.47 | 1.10 | -21.9 | |
| SB 1 | 9.25 | 1.03 | -42.1 | 2.8 |
| SB 3 | 7.68 | 0.95 | -51.9 | |

**Table 3. Human blood. Expression of IL-10 stimulated by 1 µg/ml LPS Mean and SD. N = 8**

| Product µM | IL-10 ng/ml | SD ng/ml | %Diff. vs control |
|---|---|---|---|
| Control | 0.25 | 0.07 | 0.0 |
| | | | |
| Man 3 | 0.27 | 0.05 | 6.3 |
| Man 10 | 0.32 | 0.06 | 27.6 |
| Man 30 | 0.73 | 0.08 | 187.4 |
| | | | |
| CGS 30 | 0.25 | 0.05 | -3.5 |
| CGS 100 | 0.26 | 0.06 | 1.6 |
| CGS 300 | 0.27 | 0.06 | 6.3 |
| | | | |
| ASS 3 | 0.29 | 0.05 | 12.6 |
| ASS 10 | 0.40 | 0.06 | 57.5 |
| ASS 30 | 0.90 | 0.10 | 253.5 |
| | | | |
| SB 0.3 | 0.39 | 0.06 | 53.9 |
| SB 1 | 0.48 | 0.09 | 87.4 |
| SB 3 | 0.69 | 0.07 | 170.9 |

**Table 4. Human blood. Coagulation stimulated thromboxane (TXB2) production Mean and SD. N = 8**

| Product µm | TXB2 ng/ml | SD ng/ml | %Diff. vs control |
|---|---|---|---|
| Baseline | 120 | 10 | 0.0 |
| | | | |
| Man 1 | 104 | 12 | -13.3 |
| Man 3 | 91 | 11 | -24.2 |
| Man 10 | 99 | 9 | -17.5 |
| | | | |
| CGS 10 | 124 | 28 | 3.3 |
| CGS 30 | 118 | 23 | -1.7 |
| CGS 100 | 125 | 20 | 4.2 |
| | | | |
| ASS1 | 115 | 17 | -4.2 |
| ASS 3 | 100 | 6 | -16.7 |
| ASS 10 | 108 | 4 | -10.0 |

**Table 5. Human blood: Expression of PGE2 stimulated by 100 µg/ml LPS Mean and SD. N = 8**

| Product µM | PGE2 ng/ml | SD ng/ml | %Diff. vs control | IC₅₀ µM |
|---|---|---|---|---|
| Control | 24.5 | 2.2 | 0.0 | |
| | | | | |
| Man 1 | 20.2 | 1.8 | -17.6 | |
| Man 3 | 16.4 | 1.5 | -33.1 | 12.3 |
| Man 10 | 12.6 | 1.2 | -48.6 | |
| | | | | |
| CGS 10 | 22.7 | 2.1 | -7.3 | |
| CGS 30 | 24.3 | 2.2 | -0.8 | N/A |
| CGS 100 | 23.5 | 2.1 | -4.1 | |
| | | | | |
| ASS 3 | 18.1 | 1.7 | -26.1 | |
| ASS 10 | 11.3 | 1.0 | -53.9 | 7.8 |
| ASS 30 | 6.2 | 0.6 | -74.7 | |
| | | | | |
| SB 0.3 | 18.4 | 1.7 | -24.9 | |
| SB 1 | 8.7 | 0.8 | -64.5 | 0.8 |
| SB 3 | 2.4 | 0.2 | -90.2 | |

**Table 6. Rabbit chondrocytes. Production of PGE2 stimulated by 1 ng/ml IL-1β Mean and SD. N = 8**

| Product µM | PGE2 ng/ml | SD ng/ml | %Diff. vs control | IC₅₀ µM |
|---|---|---|---|---|
| Baseline | 9 | 4 | | |
| | | | | |
| IL-1β | 188 | 77 | 0.0 | |
| | | | | |
| Man 0.1 | 144 | 12 | -23.4 | |
| Man 0.3 | 95 | 15 | -49.5 | 0.38 |
| Man 1 | 20 | 6 | -89.4 | |
| Man 3 | 11 | 5 | -94.1 | |
| | | | | |
| CGS 1 | 200 | 60 | 6.4 | |
| CGS 3 | 180 | 70 | -4.3 | N/A |
| CGS 10 | 210 | 60 | -11.7 | |
| | | | | |
| ASS 0.1 | 140 | 45 | -25.5 | |
| ASS 0.3 | 105 | 12 | -44.1 | 0.42 |
| ASS 1 | 22 | 9 | -88.3 | |

**Table 7. Rabbit cartilage explants. Percentage GAG released. Mean and SD. N = 8**

| Product µM | %GAG released | SD | %Diff. vs baseline | PC₅₀ µM |
|---|---|---|---|---|
| Baseline | 10 | 2 | 85.5 | |
| | | | | |
| IL-1β | 69 | 10 | 0.0 | |
| | | | | |
| Man 0.1 | 72 | 12 | -4.3 | |
| Man 0.3 | 62 | 17 | 10.1 | N/A |
| Man 1 | 65 | 10 | 5.8 | |
| | | | | |
| CGS 1 | 41 | 10 | 40.6 | |
| CGS 3 | 25 | 8 | 63.8 | 2.3 |
| CGS 10 | 16 | 9 | 76.8 | |
| | | | | |
| ASS 0.1 | 43 | 12 | 37.7 | |
| ASS 0.3 | 24 | 8 | 65.2 | 2.2* |
| ASS 1 | 15 | 3 | 78.3 | |
| | | | | |
| CHX | 12 | 5 | 82.6 | |

| | | | | |
|---|---|---|---|---|
| * With reference to CGS | | | | |

**Table 8. Rat. Paw oedema from zymosan. Oedema mlx24 hours Means, differences and SE. N = 10**

| Product and dose | Oedema Δmlx24h | Diff. vs. control | SE Diff. | t_{diff} | % Diff. vs. control |
|---|---|---|---|---|---|
| Control | 15.7 | 0.0 | 0.47 | 0.00 | 0 |
| | | | | | |
| Man 3 mg/kg | 15.3 | -0.4 | 0.46 | 0.85 | -3 |
| Man 10 mg/kg | 14.6 | -1.1 | 0.47 | 2.35 | -7 |
| Man 30 mg/kg | 12.9 | -2.8 | 0.45 | 6.37 | -18 |
| | | | | | |
| CGS 40 mg/kg | 15.6 | -0.1 | 0.47 | 0.22 | -1 |
| CGS 133 mg/kg | 15.4 | -0.3 | 0.47 | 0.64 | -2 |
| CGS 400 mg/kg | 15.2 | -0.6 | 0.41 | 0.47 | -4 |
| | | | | | |
| ASS 3 | 14.6 | -1.1 | 0.46 | 2.44 | -7 |
| ASS 10 | 13.4 | -2.4 | 0.44 | 5.37 | -15 |
| ASS 30 | 10.9 | -4.8 | 0.41 | 11.74 | -31 |
| | | | | | |
| CLX 10 mg/kg | 11.8 | -4.7 | 0.41 | 11.28 | -30 |

**Table 9. Rat. Analgesic activities of the test substances Means, differences and SE. N = 10**

| Product and dose | Analgesia Δgx24h | Diff. vs. control | SE Diff. | t_{diff} | % Diff. vs. control |
|---|---|---|---|---|---|
| Control | 1071 | 0.0 | 126 | 0.00 | 0 |
| | | | | | |
| Man 3 mg/kg | 931 | -140 | 118 | 1.18 | -13 |
| Man 10 mg/kg | 694 | -378 | 106 | 3.55 | -35 |
| Man 30 mg/kg | 557 | -514 | 101 | 5.11 | -48 |
| | | | | | |
| CGS 40 mg/kg | 1079 | 7 | 127 | 0.22 | 1 |
| CGS 133 mg/kg | 1012 | -59 | 123 | 0.64 | -6 |
| CGS 400 mg/kg | 1026 | -46 | 124 | 1.21 | -4 |
| | | | | | |
| ASS 3 | 923 | -148 | 118 | 2.44 | -14 |
| ASS 10 | 680 | -391 | 106 | 5.37 | -36 |
| ASS 30 | 436 | -635 | 96 | 11.74 | -59 |
| | | | | | |
| CLX 10 mg/kg | 574 | -497 | 101 | 11.28 | -46 |

## Claims

1. A pharmaceutical composition or dietary supplement or other preparation, comprising glucosamine, in the form of a simple or mixed salt thereof, in association with mangostin.

2. The composition according to claim 1, **characterised in** comprising crystalline glucosamine sulphate.

3. The composition according to claims 1 or 2, **characterised in** comprising an extract of *Garcinia mangostana* fruit containing mangostin.

4. The composition according to any of the claims 1 to 3, **characterised in** comprising a dried extract of *Garcinia mangostana* fruit with a mangostin content of 10% to 60% by weight, preferably 40% by weight.

5. The composition according to any of the claims 1 to 4, **characterised in** comprising glucosamine and mangostin in a molar ratio range comprised of between 2.5:1 and 500:1, preferably between 10:1 and 50:1, in particular 19:1.

6. The composition according to any of the claims 1 to 5, in forms suitable for oral administration, such as tablets, capsules or powders to be used as suspensions in aqueous or liquid carriers.

7. The use of a composition according to any of the claims 1 to 6, for the preparation of a medicament or a dietary supplement or another preparation useful for maintaining osteoarticular wellbeing and trophism.

8. The use of a composition according to any of the claims 1 to 6, for the preparation of a medicament or a dietary supplement or another preparation useful for the treatment of degenerative or inflammatory afflictions of the osteoarticular apparatus.

9. The use of a composition according to any of the claims 1 to 6 for the preparation of a medicament or a dietary supplement or another preparation, useful for the treatment and prevention of osteoarthritis.

10. The use of a composition according to any of the claims 1 to 6, for the preparation of a medicament or a dietary supplement or another preparation with anti-COX-2 activity.

11. The use of a composition according to any of the claims 1 to 6, for the preparation of a medicament or a dietary supplement or another preparation with anti-inflammatory and/or analgesic activity.

12. The use of a composition, for oral administration, according to any of the claims 1 to 6 for the preparation of a medicament or dietary supplement or another preparation suitable for the simultaneous administration of 200-1000 mg of glucosamine, as it is or in the form of a simple or mixed salt thereof, and mangostin at doses of between 5 and 180 mg, as it is or in the form of an extract of *Garcinia mangostina* fruit, preferably a dried extract containing 10% to 60% mangostin by weight.
